# EUROPÄISCHE PATENTSCHRIFT

(11) **EP 3 045 130 B1**
(45) Veröffentlichungstag und Bekanntmachung des Hinweises auf die Patenterteilung: **26.09.2018**
(21) Anmeldenummer: 16150902.1
(22) Anmeldetag: 12.01.2016
(51) Int. Cl.: A61B 17/80

(54) **IMPLANTAT ZUR TEMPORÄREN EPIPHYSEODESE ODER HEMIEPIPHYSEODESE**
IMPLANT FOR TEMPORARY EPIPHYSIODESIS OR HEMIEPIPHYSIODESIS
IMPLANT D'EPIPHYSIODESE OU HEMIEPIPHYSIODESE TEMPORAIRE

(30) Priorität: 14.01.2015 DE 102015000472; 02.02.2015 DE 102015001296
(43) Veröffentlichungstag der Anmeldung: 20.07.2016
(73) Patentinhaber: Königsee Implantate GmbH, 07426 Allendorf OT Aschau (DE)
(72) Erfinder: Döderlein, Leonhard, 83229 Aschau i. Chiemgau (DE); Tolksdorf, Joachim, 07426 Königsee-Rottenbach (DE); Deile, Steven, 99423 Weimar (DE); Orschler, Frank, 99084 Erfurt (DE)
(74) Vertreter: Meissner Bolte Partnerschaft mbB

(56) Entgegenhaltungen:
- EP-A1- 1 247 494
- WO-A2-02/09605
- WO-A2-2007/044391
- DE-A1-102005 042 766
- US-A1- 2006 142 767
- US-A1- 2006 155 298
- US-A1- 2008 300 637

## Beschreibung

Die Erfindung betrifft ein Implantat zur temporären Epiphyseodese oder Hemiepiphyseodese bestehend aus einem plattenförmigen, langgestreckten Körper mit mindestens zwei Öffnungen zum Aufnehmen von Knochenschrauben, wobei der plattenförmige Körper im Bereich zwischen den Öffnungen einen Schwächungsabschnitt sowie einen Durchbruch aufweist, welcher operativ auf der Epiphysenfuge liegt, gemäß Oberbegriff des Anspruchs 1.

Unter Epiphyseodese wird ein chirurgisches Verfahren verstanden, das zur zeitweisen Blockierung des Wachstums an langen Röhrenknochen oder Wirbelkörpern durch Überbrückung oder Zerstörung der Wachstumsfuge dient.

Bei der temporären Epiphyseodese wird eine vorübergehende Unterbrechung des Wachstums vorgenommen. Bei der Hemiepiphyseodese, die z.B. bei X- oder O-Beinen zur Anwendung kommt, wird nur eine Seite der Wachstumsfuge geschlossen. Durch diese einseitige Schließung kommt es zu einer Wachstumslenkung.

Häufigste operative Anwendungen betreffen die Wachstumsfugen in Knienähe, die knienahe Oberschenkelfuge und die knienahe Schienbeinfuge, welche zugleich die Wachstumsfuge des menschlichen Körpers mit der größten Wachstumspotenz darstellen.

Die Anwendung von sogenannten Blount-Klammern stellte sich über viele Jahre als das Standardverfahren zur temporären Epiphyseodese zum Zweck der Wachstumslenkung dar. Die Blount-Klammern werden quer auf die Wachstumsfuge gesetzt und verhindern das Wachstum lokal im Klammerbereich durch Kompression. Üblicherweise werden pro Seite zwei bis drei Klammern parallel zueinander genutzt. Derartige Klammern werden z.B. in der US 2006/0142767 beschrieben.

Weiterhin ist ein plattenförmiges Implantat mit der Gestalt in Form einer Acht bekannt, welches zwei Öffnungen für divergierende Knochenschrauben besitzt.

Die Schrauben sollen hierbei wie ein Drehgelenk fungieren und eine Kompression der Epiphysenfuge verhindern. Damit ist das vorbekannte Implantat in der Lage, den natürlichen Wachstumsprozess sowie die Extremitätenausrichtung zu lenken und eine invasive Osteotomie überflüssig zu machen. Angeboten und vertrieben werden derartige Platten von der Orthofix GmbH, Deutschland; siehe auch www.orthofix.de.

Aus der EP 1 931 287 B1 ist ein Knochenausrichtungsimplantat vorbekannt, welches zwei oder mehrere Befestigungselemente zum Fixieren an einem Knochenabschnitt besitzt, wobei die Befestigungselemente durch Verbindungsglieder eine feste Basis bilden. Bei einer Ausführungsform des vorbekannten Knochenausrichtungsimplantats ist eine Verformbarkeit gegeben und es ist mindestens eines der Befestigungselemente mit einem Widerhaken versehen.

Eine spezielle Ausführungsform eines Implantats zur temporären Epiphyseodese nach EP 1 931 287 B1 umfasst zwischen zwei Öffnungen zum Aufnehmen von Knochenschrauben einen Schwächungsabschnitt und einen Durchbruch. Sowohl der Schwächungsabschnitt als auch der Durchbruch liegen operativ auf der Epiphysenfuge. Der Schwächungsabschnitt ist gemäß der Lehre nach EP 1 931 287 B1 in Form von die Plattenbreite schwächenden seitlichen U-förmigen Ausnehmungen realisiert.

Aus der DE 10 2005 042 766 A1 ist eine Knochenplatte bekannt, mit Öffnungen die zwischen Gewindeabschnitten gewindefreie Abschnitte aufweist. Aus der US2006/0155298 A1 ist eine Knochenplatte bekannt, mit einer Nut, die auf der Plattenoberseite einen Durchbruch schneidet und senkrecht zur Plattenlängsachse verläuft. Aus der EP 1 247 494 A1 ist eine Knochenplatte bekannt, mit Öffnungen und dazwischen auf der Unterseite seitliche Unterschneidungen.

Es hat sich gezeigt, dass im Vergleich zwischen Knochenausrichtungsimplantaten und den erwähnten Blount-Klammern erhebliche Unterschiede bezüglich der Operationszeit, und zwar sowohl hinsichtlich der Dauer der Implantation als auch der Materialentfernung bestehen. Bei plattenförmigen Implantaten war insgesamt eine kürzere Operationszeit und eine Zeiteinsparung bei der Implantatentfernung zu verzeichnen.

Der Nachteil bei der Anwendung bekannter Platten liegt allerdings darin, dass zum korrekten Ausrichten und Vorfixieren am Knochen der Einsatz von sogenannten Moskitoklemmen notwendig ist. Weiterhin führen die Öffnungen bekannter Platten zur Aufnahme divergierender Schrauben dazu, dass eine Steuerung des Wachstums des Knochens nicht möglich ist und hier mehr oder weniger zufällige Ergebnisse oder lediglich auf umfangreichen Erfahrungen basierende Wachstumsbeeinflussungen in reproduzierbarer Weise ermöglicht werden.

Aus dem Vorgenannten ist es daher Aufgabe der Erfindung, ein weiterentwickeltes Implantat zur temporären Epiphyseodese oder Hemiepiphyseodese auf der Basis eines plattenförmigen, langgestreckten Körpers mit Öffnungen zum Aufnehmen von Knochenschrauben anzugeben, welches variabel in der Anwendung und Indikation ist und das sehr einfach ohne Schädigung der Epiphysenfuge entfernbar ist.

Die Lösung der Aufgabe der Erfindung erfolgt mit einem Implantat zur temporären Epiphyseodese oder Hemipiphyseodese gemäß der Merkmalskombination nach Anspruch 1, wobei die Unteransprüche mindestens zweckmäßige Ausgestaltungen und Weiterbildungen darstellen.

Es wird demnach von einem Implantat zur temporären Epiphyseodese oder Hemiepiphyseodese, bestehend aus einem plattenförmigen, langgestreckten Körper mit mindestens zwei Öffnungen zum Aufnehmen von Knochenschrauben ausgegangen, wobei der plattenförmigen Körper im Bereich zwischen den Öffnungen, die zur Aufnahme von Knochenschrauben dienen, einen Schwächungsabschnitt sowie einen Durchbruch aufweist. Der Schwächungsabschnitt und der Durchbruch liegen operativ im Wesentlichen auf der Epiphysenfuge.

Erfindungsgemäß sind die Öffnungen der Platte mit jeweils einem mindestens ein- oder mehrgängigen Gewinde versehen, wobei das Gewinde derart segmentiert ist, dass innenumfangsseitig zwischen Gewindeabschnitten gewindefreie Abschnitte verbleiben.

Das Gewinde stellt hierbei ein Innengewinde dar, welches so realisiert ist, dass selbiges komplementär zu einem Außengewinde auf einer diesbezüglich ausgebildeten Knochenschraube bzw. einem Knochenschraubenkopf ist.

Weiterhin befinden sich erfindungsgemäß an den Enden des plattenförmigen Körpers jeweils mindestens eine Bohrung zur Aufnahme eines Kirschner-Drahts.

Der Schwächungsabschnitt ist erfindungsgemäß als oberflächenseitige, d.h. an der Plattenoberseite befindliche Nut ausgebildet, welche den Durchbruch schneidet und im Wesentlichen senkrecht zur Plattenlängsachse verläuft.

Die Plattenunterseite ist erfindungsgemäß im Bereich zwischen den Öffnungen mit segmentierten Gewinden mit einer Unterschneidung versehen.

Diese Unterschneidung verläuft im Wesentlichen entlang der Plattenlängsunterseiten und an den Außenkanten. Mit anderen Worten ist nicht die gesamte Plattenunterseite mit einer Unterschneidung versehen, sondern lediglich ein bestimmter Breitenabschnitt in Längsrichtung verfügt über die vorerwähnte Unterschneidung.

Bei einer Ausgestaltung der Erfindung weist die Plattenunterseite an den gegenüberliegenden Enden mindestens jeweils einen krallenförmigen Fortsatz auf.

Der krallenförmige Fortsatz kann als Einfachkralle oder doppel- bzw. mehrfach kralfenförmiger Haken ausgeführt sein.

Wie bereits erwähnt, verläuft die Unterschneidung bzw. die beidseitigen Unterschneidungen ausgehend von der jeweiligen Plattenlängsseite in Richtung Durchbruch, erreicht diesen jedoch nicht.

Die Unterschneidung ist bevorzugt als ein Abschnitt mit Materialabtrag auf der Plattenunterseite realisierbar.

Die Unterschneidung ist wahlweise auf beiden Plattenlängsseiten oder nur auf einer Plattenlängsseite ausgeführt.

Bei einer Weiterbildung der Erfindung ist der plattenförmige Körper im Bereich der Nut tailliert ausgebildet, d.h. die Breite der Platte nimmt in Richtung der Lage der Nut kontinuierlich bis zu einem vorgegebenen Endwert ab.

Bei einer Ausführungsform der Erfindung besitzt der plattenförmige Körper abgerundete und/oder mit einer Fase versehene Plattenenden bzw. Plattenoberflächen, so dass eine Verletzung von Weichteilen weitgehend vermeidbar ist.

Der jeweilige krallenförmige Fortsatz ist auf der Plattenlängsachse der Plattenunterseite befindlich und ist als auslaufende, zur Plattenunterseite gebogene Plattenspitze realisierbar.

Die Öffnungen mit Gewinde dienen, wie bereits dargelegt, einerseits der Aufnahme von Standard-Knochenschrauben mit sphärischem Schraubenkopf, aber auch der Aufnahme von Knochenschrauben mit Gewindekopf. Hierdurch ist es möglich, die Platte sehr variabel anzuwenden. Kommen Standardschrauben zum Einsatz, findet ein kontrolliertes reduzierendes Knochenwachstum statt. Bei der Anwendung von Knochenschrauben mit Gewindekopf ergeben sich variabel winkelstabile Schraubverbindungen, so dass das Wachstum des Knochens vollständig gebremst werden kann. Darüber hinaus ermöglichen variabel winkelstabile Schrauben ein schräges Einbringen derselben in die Epiphyse, was weitere Indikationen ermöglicht.

Erfindungsgemäß weist die Platte maximal eine Plattendicke von 2 mm auf und ist damit deutlich flacher als bekannte Produkte realisiert, was insofern einen wesentlichen Vorteil darstellt, da im üblichen Applikationsbereich der Platte wenig Weichteilgewebe vorhanden ist.

Die erfindungsgemäße oberflächenseitige Nut bzw. Einkerbung ermöglicht ein kontrolliertes Anformen der Platte an die gegebene Anatomie des Patienten. Eine unerwünschte Verformung der Öffnungen zum Aufnehmen der Knochenschrauben ist ausgeschlossen.

Die an den Enden des Plattenkörpers vorgesehenen Bohrungen sind als periphere Kirschner-Drahtlöcher realisiert und dienen dem korrekten Ausrichten und Vorfixieren der Platte am Knochen.

Die krallenförmigen Fortsätze ermöglichen nicht nur ein Vorfixieren der Platten, sondern dienen der Minimierung der Plattenauflagenfläche, um eine Periostschädigung zu vermeiden und gleichzeitig die Bildung einer unerwünschten Knochenbrücke zu verhindern.

Durch die seitlichen Unterschneidungen ist eine sehr einfache Entfernung des Implantats möglich, da nur ein sehr flaches Instrument unter die Platte geführt werden muss, um selbige vom Knochen abzuheben. Eine schonende Entfernung der Platte ist wesentlich, um eine Schädigung der Epiphysenfuge zu vermeiden. Darüber hinaus verhindert bzw. verringert die Ausbildung einer seitlichen Unterschneidung das Umwachsen der Platte mit Knochengewebe, was ebenfalls ein Beitrag zum schonenden Plattenentfernen ist.

Es sei an dieser Stelle dargelegt, dass das erfindungsgemäße Implantat selbstverständlich auch drei, vier oder mehrere Öffnungen zum Aufnehmen von Knochenschrauben besitzen kann, wobei die Möglichkeit umfasst ist, bei Ausbildung einer breiteren Platte Öffnungen zum Aufnehmen von Knochenschrauben paarweise benachbart auszubilden.

Die Erfindung soll nachstehend anhand von Ausführungsbeispielen sowie unter Zuhilfenahme von Figuren näher erläutert werden.

Hierbei zeigen:
- Fig. 1a) bis 1h): Darstellungen eines Implantates zur temporären Epiphyseodese oder Hemiepiphyseodese in taillierter Gestalt mit Öffnungen zum Aufnehmen von Knochenschrauben in Form von segmentierten Gewinden;
- Fig. 2a) bis 2h): ein Implantat ähnlich wie in der Fig. 1 gezeigt, jedoch mit krallenförmigen Fortsätzen an den gegenüberliegenden Unterseiten des langgestreckten plattenförmigen Körpers;
- Fig. 3a) bis 3h): Darstellungen eines Implantats zur temporären Epiphyseodese oder Hemiepiphyseodese in nicht taillierter Ausführungsform, jedoch wiederum mit Gewinden in den Öffnungen zur Aufnahme von Knochenschrauben und
- Fig. 4a) bis 4h): Darstellungen einer Platte ähnlich derjenigen nach Fig. 3, jedoch mit krallenförmigen Fortsätzen an den gegenüberliegenden Enden der Plattenunterseite.

In den Figuren ist mit jeweils a) die Draufsicht eines Implantates, mit b) die Unteransicht eines Implantates, mit c) und d) Längsseitenansichten und mit e) und f) Seitenansichten bezeichnet.

Eine perspektivische Unterseitenansicht ist mit g) und eine perspektivische Draufsicht mit h) versehen.

Die in den Figuren gezeigten Implantate zur temporären Epiphyseodese oder Hemiepiphyseodese bestehen aus einem plattenförmigen, langgestreckten Körper mit in den Beispielen gezeigten Öffnungen 2 und 3 zum Aufnehmen von nicht dargestellten Knochenschrauben.

Quasi in der Plattenmitte bzw. im Schwerpunkt der Platten befindet sich ein Durchbruch 4, welcher der Aufnahme eines Stiftes dient, der während der Operation in die Epiphysenfuge eingedrückt wird derart, dass das Implantat dann operativ auf der Epiphysenfuge liegt.

Wie aus den Figuren ersichtlich, sind die Öffnungen 2 und 3 als mindestens ein- oder mehrgängiges Gewinde realisiert, wobei das Gewinde derart segmentiert ist, dass innenumfangsseitig zwischen Gewindeabschnitten 5 gewindefreie Abschnitte 6 verbleiben.

Weiterhin ist an den Enden des plattenförmigen Körpers 1 jeweils mittig eine Bohrung 7 zur Aufnahme eines nicht gezeigten Kirschner-Drahts vorgesehen.

Die Platte weist darüber hinaus einen Schwächungsabschnitt auf, der als Nut 8 auf der Plattenoberseite realisiert ist, wobei die Nut 8 den Durchbruch 4 schneidet und im Wesentlichen senkrecht zur Plattenlängsachse verläuft.

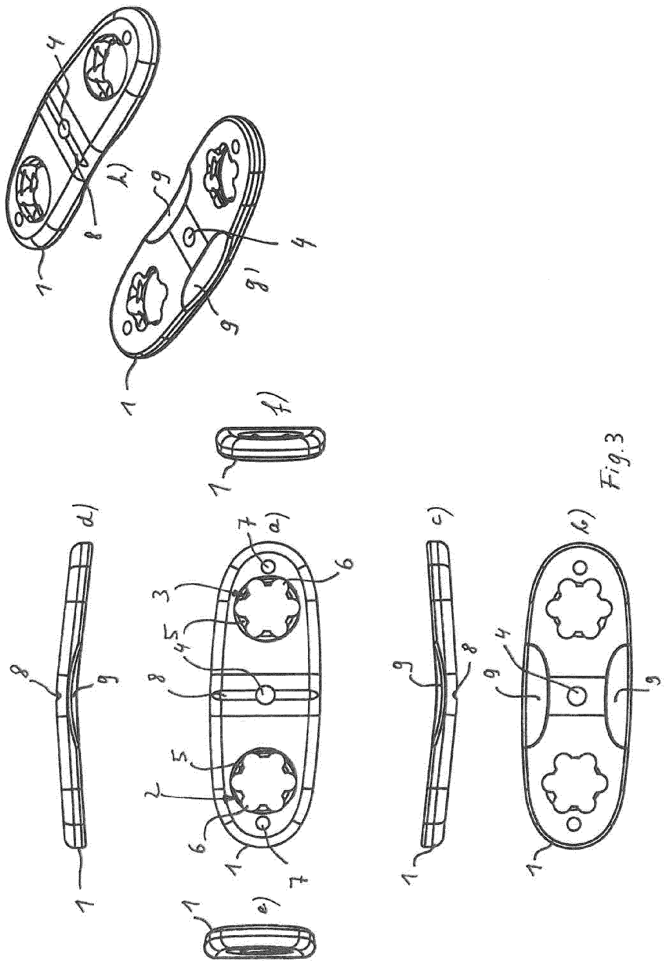

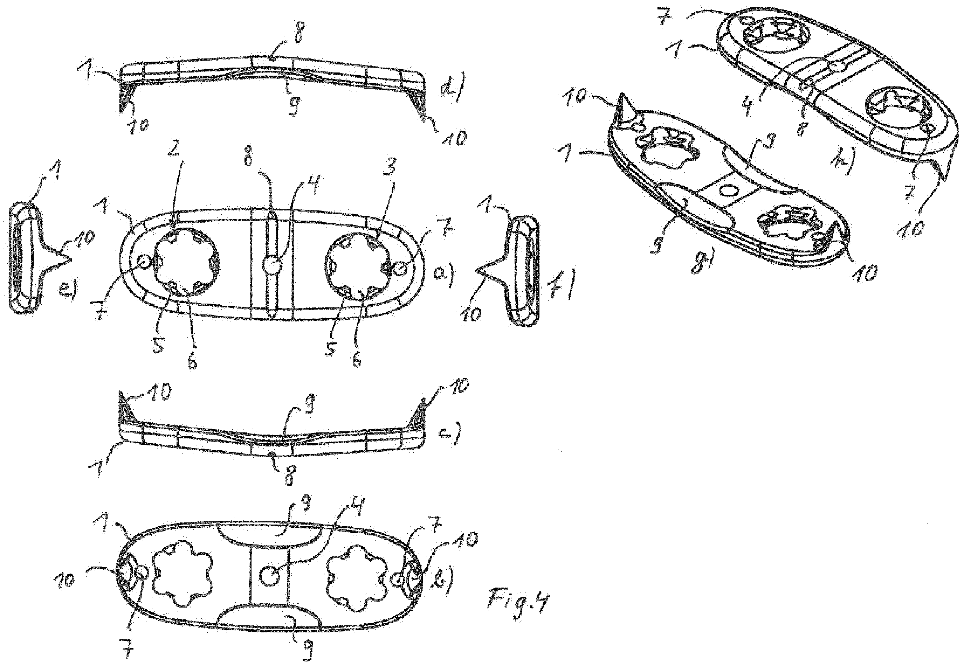

Die Plattenunterseite weist eine, insbesondere in den Fig. 3 und 4 erkennbare Unterschneidung 9 auf.

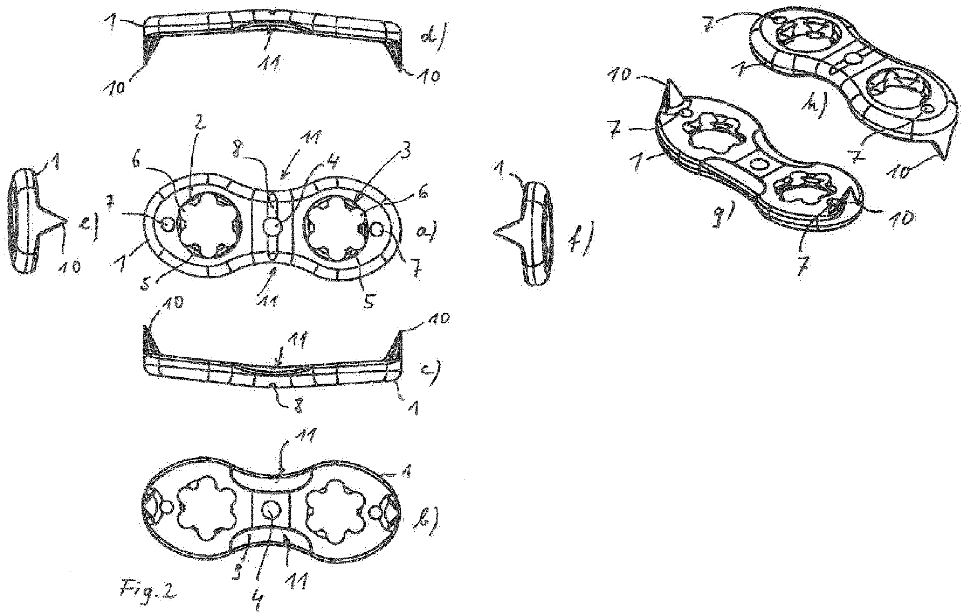

Bei der Ausführungsform der Platte bzw. des Implantats nach den Fig. 2 und 4 besitzt die Plattenunterseite an den gegenüberliegenden Enden jeweils mindestens einen krallenförmigen Fortsatz 10.

Die Unterschneidung 9 ist ausgehend von der jeweiligen Plattenlängsunterseite in Richtung Durchbruch verlaufend ausgebildet, erreicht den Durchbruch jedoch nicht. Mit anderen Worten ist die Unterschneidung 9 vorteilhafterweise nur über einen schmalen Abschnitt entlang der Unterseite und der betreffenden Außenkante der Platte ausgebildet.

Die Unterschneidung 9 wird durch einen Materialabtrag, z.B. durch Fräsen oder Schleifen auf der Plattenunterseite ausgebildet und dient zum einen der Minimierung der Auflagefläche bezogen auf den Knochen und ermöglicht zum anderen eine einfache Entfernung des Implantats durch Einfügen eines flachen Instruments unter die Platte, ohne wesentliche Schäden zu verursachen.

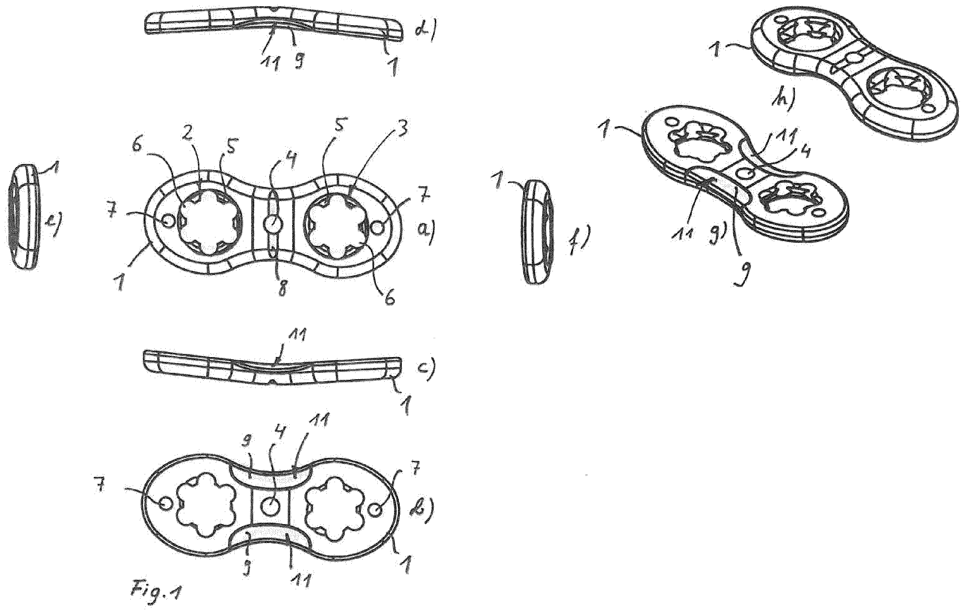

Der plattenförmige Körper kann, wie in den Fig. 1 und 2 gezeigt, im Bereich der Nut 8 seitlich tailliert (Bezugszeichen 11) ausgebildet werden.

Der plattenförmige Körper 1 kann insgesamt über eine abgerundete Oberflächenstruktur verfügen bzw. eine umlaufende Fase oder mindestens abgerundete, mit einer Fase versehene Plattenenden besitzen.

Wie aus den Fig. 2 und 4 ersichtlich wird, ist der jeweilige krallenförmige Fortsatz 10 quasi auf der Plattenlängsachse befindlich und ist als auslaufende, zur Plattenunterseite gebogene Plattenspitze realisierbar.

Die Dicke der beispielhaft gezeigten Platten beträgt maximal 2 mm, was einen Vorteil insofern darstellt, als dass im üblichen Applikationsbereich wenig Weichteilgewebe vorhanden ist.

## Patentansprüche

1. Implantat zur temporären Epiphyseodese oder Hemiepiphyseodese, bestehend aus einem plattenförmigen, langgestreckten Körper (1) mit mindestens zwei Öffnungen (2; 3) zum Aufnehmen von Knochenschrauben, wobei der plattenförmige Körper (1) im Bereich zwischen den Öffnungen (2; 3) einen Schwächungsabschnitt sowie einen Durchbruch (4) aufweist, welcher operativ auf der Epiphysenfuge liegt,
**dadurch gekennzeichnet, dass**
die Öffnungen (2; 3) mit einem mindestens eingängigen Gewinde versehen sind, wobei das Gewinde derart segmentiert ist, dass innenumfangsseitig zwischen Gewindeabschnitten (5) gewindefreie Abschnitte (6) verbleiben, weiterhin an den Enden des plattenförmigen Körpers (1) jeweils mindestens eine Bohrung (7) zur Aufnahme eines Kirschner-Drahts vorgesehen ist,
der Schwächungsabschnitt als Nut (8) ausgebildet ist, welche auf der Plattenoberseite den Durchbruch (4) schneidet und im Wesentlichen senkrecht zur Plattenlängsachse verläuft sowie die Plattenunterseite im Bereich zwischen den Öffnungen (2; 3) mit segmentiertem Gewinde mindestens eine seitliche Unterschneidung (9) besitzt.

2. Implantat nach Anspruch 1,
**dadurch gekennzeichnet, dass**
die Plattenunterseite an den gegenüberliegenden Enden der Platte mindestens jeweils einen krallenförmigen Fortsatz (10) aufweist.

3. Implantat nach Anspruch 1 oder 2,
**dadurch gekennzeichnet, dass**
die Unterschneidung (9) ausgehend von der jeweiligen Plattenlängsunterseite in Richtung Durchbruch (4) verläuft, diesen jedoch nicht erreicht.

4. Implantat nach einem der vorangegangenen Ansprüche,
**dadurch gekennzeichnet, dass**
die Unterschneidung (9) als ein Abschnitt mit Materialabtrag auf der Plattenunterseite ausgebildet ist.

5. Implantat nach einem der vorangegangenen Ansprüche,
**dadurch gekennzeichnet, dass**
die Unterschneidung (9) auf beiden oder wahlweise auf einer der Plattenlängsseiten realisiert ist.

6. Implantat nach einem der vorangegangenen Ansprüche,
**dadurch gekennzeichnet, dass**
der plattenförmige Körper (1) im Bereich der Nut (8) tailliert (11) ausgebildet ist.

7. Implantat nach einem der vorangegangenen Ansprüche,
**dadurch gekennzeichnet, dass**
der plattenförmige Körper (1) abgerundete und/oder mit einer Fase versehene Plattenenden besitzt.

8. Implantat nach einem der Ansprüche 2 bis 7,
**dadurch gekennzeichnet, dass**
der jeweilige krallenförmige Fortsatz (10) auf der Plattenlängsachse befindlich und als auslaufende, zur Plattenunterseite gebogene Plattenspitze realisiert ist.

9. Implantat nach einem der vorangegangenen Ansprüche,
**dadurch gekennzeichnet, dass**
die Öffnungen (2; 3) mit Gewinde zur Aufnahme von sowohl Standard-Knochenschrauben mit sphärischem Kopf als auch Knochenschrauben mit Gewindekopf ausgebildet sind.

10. Implantat nach einem der vorangegangenen Ansprüche,
**dadurch gekennzeichnet, dass**
die Plattendicke maximal 2 mm beträgt.

## Claims

1. Implant for temporary epiphysiodesis or hemiepiphysiodesis,
consisting of a plate-shaped elongated body (1) with at least two apertures (2; 3) for receiving bone screws, the plate-shaped body (1) comprising in the area between the apertures (2; 3) a weakening portion and an opening (4) which is surgically located on the epiphyseal plate, **characterized in that**
the apertures (2; 3) are provided with an at least single-start thread, the thread being segmented so that there remain at the inner circumference between the threaded portions (5) unthreaded portions (6),
wherein furthermore there is in each case at least one bore (7) provided at the ends of the plate-shaped body (1) for receiving a Kirschner wire, the weakening portion being formed as a groove (8) which intersects the opening (4) on the upper side of the plate (4) and which is oriented substantially perpendicular to the longitudinal axis of the plate, and the underside of the plate has at least one lateral undercut (9) in the area between the apertures (2; 3) with segmented thread.

2. Implant according to Claim 1, **characterized in that** the underside of the plate has at least one claw-shaped extension (10) at each of the opposite ends of the plate.

3. Implant according to Claim 1 or 2, **characterized in that** the undercut (9) runs from the respective longitudinal underside of the plate in the direction of the opening (4), but does not reach it.

4. Implant according to one of the preceding claims, **characterized in that** the undercut (9) is formed as a segment with material removal on the underside of the plate.

5. Implant according to one of the preceding claims, **characterized in that** the undercut (9) is realized on both or optionally on one of the longitudinal sides of the plate.

6. Implant according to one of the preceding claims, **characterized in that** the plate-shaped body (1) is formed waisted (11) in the region of the groove (8).

7. Implant according to one of the preceding claims, **characterized in that** the plate-shaped body (1) has rounded and/or chamfered plate ends.

8. Implant according to one of the Claims 2 to 7, **characterized in that** the respective claw-shaped extension (10) is arranged on the longitudinal axis of the plate and is realized as a tapered plate tip bent to the underside of the plate.

9. Implant according to one of the preceding claims, **characterized in that** the apertures (2; 3) are formed with a thread to accommodate both standard bone screws with spherical head and bone screws with threaded head.

10. Implant according to one of the preceding claims, **characterized in that** the maximum plate thickness is 2 mm.

## Revendications

1. Implant pour l'épiphysiodèse ou l'hémiépiphysiodèse temporaire, constitué par un corps allongé en forme de plaque (1) pourvu d'au moins deux ouvertures (2 ; 3) pour loger des vis à os, le corps en forme de plaque (1) présentant dans la zone entre les ouvertures (2 ; 3) une portion d'affaiblissement ainsi qu'une traversée (4) qui se situe chirurgicalement sur la jointure d'épiphyse,
**caractérisé en ce que**
les ouvertures (2 ; 3) sont pourvues d'un taraudage présentant au moins un pas simple, le taraudage étant segmenté de telle sorte que des portions (6) dépourvues de taraudage demeurent du côté périphérie intérieure entre les portions taraudées (5), et aux extrémités du corps en forme de plaque (1) est prévu respectivement au moins un perçage (7) pour loger une broche de Kirschner, la portion d'affaiblissement est réalisée sous forme de gorge (8) qui croise la traversée (4) sur la face supérieure de la plaque et qui s'étend sensiblement perpendiculairement à l'axe longitudinal de la plaque, et la face inférieure de la plaque possède au moins une contre-dépouille latérale (9) dans la zone entre les ouvertures (2 ; 3) pourvue du taraudage segmenté.

2. Implant selon la revendication 1,
**caractérisé en ce que**
aux extrémités opposées de la plaque, la face inférieure de la plaque présente au moins un prolongement respectif (10) en forme de griffe.

3. Implant selon la revendication 1 ou 2,
**caractérisé en ce que**
la contre-dépouille (9) s'étend à partir de la face inférieure longitudinale respective de la plaque en direction de la traversée (4), cependant sans l'atteindre.

4. Implant selon l'une des revendications précédentes,
**caractérisé en ce que**
la contre-dépouille (9) est réalisée sous la forme d'une portion réalisée par enlèvement de matière sur la face inférieure de la plaque.

5. Implant selon l'une des revendications précédentes,
**caractérisé en ce que**
la contre-dépouille (9) est réalisée sur les deux faces longitudinales de la plaque ou au choix sur l'une de celles-ci.

6. Implant selon l'une des revendications précédentes,
**caractérisé en ce que**
le corps en forme de plaque (1) est réalisé avec un rétrécissement (11) dans la zone de la gorge (8).

7. Implant selon l'une des revendications précédentes,
**caractérisé en ce que**
le corps en forme de plaque (1) possède des extrémités de plaque arrondies et/ou pourvues d'un chanfrein.

8. Implant selon l'une des revendications 2 à 7,
**caractérisé en ce que**
le prolongement respectif (10) en forme de griffe est réalisé de manière à se situer sur l'axe longitudinal de la plaque et sous forme de pointe de plaque divergeante incurvée vers la face inférieure de la plaque.

9. Implant selon l'une des revendications précédentes,
**caractérisé en ce que**
les ouvertures (2 ; 3) sont réalisées avec un taraudage pour loger aussi bien des vis à os standard à tête sphérique que des vis à os à tête filetée.

10. Implant selon l'une des revendications précédentes,
**caractérisé en ce que**
l'épaisseur de plaque est au maximum de 2 mm.
